Europäisches Patentamt

European Patent Office

Office européen des brevets

(11) Publication number: **0 177 191**
**B1**

(12) **EUROPEAN PATENT SPECIFICATION**

(45) Date of publication of patent specification: **22.11.90**

(21) Application number: **85306272.7**

(22) Date of filing: **04.09.85**

(51) Int. Cl.⁵: **G 01 N 33/537,**
**G 01 N 33/74, G 01 N 33/577,**
**G 01 N 33/563,**
**G 01 N 33/538**

(54) Methods of assay.

(30) Priority: **05.09.84 GB 8422452**

(43) Date of publication of application:
**09.04.86 Bulletin 86/15**

(45) Publication of the grant of the patent:
**22.11.90 Bulletin 90/47**

(84) Designated Contracting States:
**AT BE CH DE FR GB IT LI NL SE**

(56) References cited:
**EP-A-0 005 271     DE-A-2 952 373**
**EP-A-0 028 132     DE-A-3 235 516**
**EP-A-0 055 868     GB-A-2 013 211**
**EP-A-0 064 318     GB-A-2 014 162**
**EP-A-0 113 075     GB-A-2 084 317**

**CLINICAL CHEMISTRY, vol. 30, no. 9, September
1984, pages 1457-1461, Winston-Salem, NC, US;
S.J. RATTLE et al.: "New separation method for
monoclonal immunoradiometric assays and its
application to assays for thyrotropin and human
choriogonadotropin"**

(73) Proprietor: **SERONO DIAGNOSTICS PARTNERS
(A MASSACHUSETTS LIMITED PARTNERSHIP)**
**Exchange Place, 37th Floor**
**Boston Massachusetts 02109 (US)**

(72) Inventor: **Allen, Gerald John**
**40 Chertsey Road**
**Windlesham Surrey (GB)**

(74) Representative: **Woodman, Derek et al**
**Frank B. Dehn & Co. European Patent Attorneys**
**Imperial House 15-19 Kingsway**
**London WC2B 6UZ (GB)**

## EP 0 177 191 B1

**Description**

The present invention relates to 1-site immunometric assays and related assays and to kits for carrying out such assays.

1-Site immunometric assays, for example for measuring components of samples of biological fluids, are well described in the literature. The basis of such assays is that during the assay, a labelled specific binding partner (namely an antibody) for the species under assay (the species under assay being hereinafter referred to as "the ligand") becomes distributed between the ligand and a ligand analogue. (The term "ligand analogue" as used herein means a species which will bind specifically to the same specific binding partner as will the ligand and includes within its scope ligand molecules distinguishable from the ligand itself). A fixed, known quantity of both ligand analogue and labelled antibody are employed, so that the binding of labelled antibody to ligand analogue is inversely related to the amount of ligand in the sample. By quantifying the proportion of labelled antibody that becomes bound to ligand analogue, the level of ligand in the sample can be determined. Such assays can be performed with a simultaneous incubation of sample, ligand analogue and labelled antibody (when a competitive reaction occurs), or sequentially, whereby there is an initial reaction of ligand from the sample and labelled antibody, followed by reaction with ligand analogue.

Labels which have been employed include radioactive atoms (e.g. isotopes such as $^{125}$I), enzymes and fluorescent molecules.

Conventionally, that portion of the labelled antibody which becomes bound to the ligand analogue is determined by coupling the ligand analogue to a solid phase prior to the assay, and at the completion of the assay physically separating the solid phase from the other assay reactants and quantifying the amount of label which has become bound to it. The solid phase material is generally chosen to facilitate such a separation; thus, for example, solid phase materials which have been employed include particles which may be removed by centrifugation, magnetic separation etc., e.g. finely divided inert particles or beads. Suitable magnetic particles are described in "Immunoassays for Clinical Chemistry" (Ed. Hunter & Corrie, Churchill Livingstone, Edinburgh (1983)) pp. 147—162; for example, particles of cellulose composite containing $Fe_3O_4$ may be used.

Although this method offers many advantages over saturation assays, the need for a solid phase ligand analogue component presents a serious drawback. In particular, the component may be difficult or expensive to produce, and a different solid phase component will be required for each ligand assayed. In addition, the coupling of ligand analogue to the solid phase may hinder the subsequent specific binding reaction, or adversely affect the affinity or specificity of the interaction. Furthermore, the solid phase component will generally be less mobile than the ligand under assay, which may delay attainment of equilibrium (resulting in long assay times) and/or necessitate stirring of the assay medium.

GB—2084317 discloses a 1-site enzyme-linked immunometric assay method whereby the disadvantages mentioned above of coupling the ligand analogue directly to the solid phase are avoided. This method involves the use of a ligand analogue conjugated with a reagent X, a solid phase carrying a binding partner specific for reagent X, a first antibody ($Ab_1$) capable of binding to both the ligand and the ligand analogue and an enzyme-labelled second antibody ($Ab_2$*) capable of binding to the first antibody. It is particularly convenient for $Ab_2$ to be a polyclonal antibody specific for the Fc fraction of the immunoglobulin of the animal chosen for producing $Ab_1$. Such assays are not ideal, however, since either the ligand and ligand analogue must react with a very high molecular weight preformed $Ab_1$—$Ab_2$* complex (resulting in slower reaction kinetics and/or steric hindrance problems) or an additional assay step is necessitated, $Ab_2$* being added to the assay system after $Ab_1$.

We have now devised a method of assay applicable to a wide range of ligand-specific binding partner pairs wherein a ligand analogue conjugated with a reagent X is employed together with a solid phase carrying a binding partner specific for reagent X. In contrast to the technique described in GB—2084317, in the technique of the present invention the specific binding partner for the ligand is directly conjugated with a label and is referred to herein as a directly labelled specific binding partner.

According to one feature of the present invention, we provide a method of assay of a ligand in a sample, which comprises incubating, simultaneously or in sequence,

(a) the sample (containing the analyte ligand),

(b) a specific binding partner to the ligand, the said specific binding partner being directly labelled and not being part of a complex with a labelled specific binding partner therefor and

(c) a ligand analogue as herein defined, conjugated with a reagent X (the said reagent not being present as a free reagent in the mixture);

separating, after a suitable incubation period, the portion containing component (c) from the mixture by means of a solid phase carrying a binding partner specific for reagent X; and determining the extent of complexing between components (b) and (c) by measurement of the label.

Examples of ligands which may be assayed using techniques according to the invention, together with an indication of a suitable specific binding partner in each case, are shown in Table I below:

2

TABLE I

| Ligand | Specific binding partner |
|---|---|
| Antigen | antibody |
| antibody | antigen |
| hormone | hormone receptor |
| hormone receptor | hormone |
| polynucleotide strand | complementary polynucleotide strand |
| avidin | biotin |
| biotin | avidin |
| protein A | immunoglobulin |
| immunoglobulin | protein A |
| enzyme | enzyme cofactor (substrate) |
| enzyme cofactor (substrate) | enzyme |
| lectin | specific carbohydrate |
| carbohydrate | specific lectin |

The term "antigen" as used herein will be understood to include both permanently antigenic species (for example, proteins, peptide hormones, bacteria, bacteria fragments, cells, cell fragments and viruses) and haptens which may be rendered antigenic under suitable conditions (including narcotics, hypnotics, analgesics, cardiovascular drugs, vitamins, non-peptide hormones and metabolites thereof, antibiotics, pesticides and sugars).

It will be understood that the term "antibody" as used hereinafter includes within its scope
a) any of the various classes or sub-classes of immunoglobulin, e.g. IgG, IgM, derived from any of the animals conventionally used, e.g. sheep, rabbits, goats or mice,
b) monoclonal antibodies,
c) intact molecules or "fragments" of antibodies, monoclonal or polyclonal, the fragments being those which contain the binding region of the antibody, i.e. fragments devoid of the Fc portion (e.g., Fab, Fab', F(ab')$_2$) or the so-called "half-molecule" fragments obtained by reductive cleavage of the disulphide bonds connecting the heavy chain components in the intact antibody.

The method of preparation of fragments of antibodies is well known in the art and will not be described herein.

The technique of the present invention is particularly applicable to the assay of haptens including, for example, non-peptide hormones and metabolites thereof. Examples of non-peptide hormones which may be assayed by a method of the invention include steroid hormones, e.g. oestradiol, cortisol, progesterone and testosterone and thyroid hormones such as thyroxine and triiodothyronine.

The binding partner specific for reagent X may, for example, be an antibody raised to the said reagent X. Alternatively the reagent X and the binding partner specific therefor may consist of other binding pairs, e.g. avidin/biotin.

The assay may be comnpleted with reference to calibration data.

The label and the solid phase used may be selected from labels and solid phases conventionally used in the art of specific binding assays. Methods of incorporating labels into specific binding partners and measuring labels are well-known in the art.

If the ligand to be assayed is an antigen or hapten, component (b) may, for example, conveniently be an antibody capable of binding the sample ligand conjugated with an enzyme, for example β-galactosidase or alkaline phosphatase, by means of a heterobifunctional reagent [see, for example, Ishikawa et al., in J. Immunoassay 4, 209—327 (1983)].

The solid phase carrying a binding partner specific for reagent X may be introduced into the mixture before or after completion of the incubation, as desired. In particular, it may prove advantageous to add the solid phase with the three components (a), (b) and (c), thereby simplifying the assay protocol; however care would be required in that case to ensure that the attainment of equilibrium was not adversely affected. Alternatively, the solid phase may be prereacted with component (c) prior to the assay, if desired. In this case, too, care would be required to ensure that the attainment of equilibrium was not affected. Conveniently, the solid phase may comprise magnetisable particles, e.g. magnetisable cellulose particles (see Forrest and Rattle, "Magnetic Particle Radio Immunoassay", in "Immunoassays for Clinical Chemistry", p. 147—162, Ed. Hunter and Corrie, Churchill Livingstone, Edinburgh (1982)).

The reagent X may, for example, be selected from fluorescein isothiocyanate (FITC), rhodamine isothiocyanate, 2,4-dinitrofluorobenzene, phenyl isothiocyanate and dansyl chloride. When reagent X is FITC, the binding partner specific for reagent X on the solid phase may be anti-FITC antibody covalently linked to the solid support. The antiserum may be prepared in conventional manner, for example by immunising sheep with FITC conjugated to keyhole limpet haemocyanin. Coupling of the antiserum to the solid support may for example be effected using the method of Axen et al (Nature 214, 1302—1304 (1967)).

It may be found, in practice, that steric interference between component (b) of the assay mixture and

3

the solid phase may prevent simultaneous binding of both receptors and may lead to inefficient separation. This problem, if it occurs, can be overcome by using a "spacer" between reagent X and the ligand analogue in component (c). Such a spacer may, for example, be a bifunctional molecule or group of sufficient size to ensure that the solid phase does not interfere with component (b). Thus, spacers may be selected from the following: proteins, e.g. bovine serum albumin (BSA), peptides, oligosaccharides, polysaccharides, synthetic polymers etc. For example, to assay the steroid hormone oestradiol by a method according to the present invention, an oestradiol 17B-hemisuccinate BSA-FITC conjugate (commercial product of Sigma, U.K.) may be conveniently employed as component (c) and component (b) may be a directly labelled antibody capable of binding oestradiol either alone or in component (c).

Using this improvement, a reduction in assay incubation time may be achievable. In addition, the method of the present invention enables stirring of the assay medium to be avoided, thereby simplifying the apparatus. If desired the apparatus may be automated, e.g. to enable the components to be added and removed in a preset sequence and over a preset time-scale.

According to a further feature of the present invention, we provide kits of reagents and apparatus for carrying out a method of assay as hereinbefore defined. Such kits may, for example, comprise components (b) and (c) and a solid phase carrying a binding partner specific for reagent X. Desirably, reagent X will be the same in components (c) for use in a wide range of assays, so that the solid phase component will be the same in all these cases.

The following non-limiting Examples are intended to illustrate further the present invention:

Example 1
Enzyme immunoassay of thyroxine (T4)

In this assay, T4 in the sample competes with the link ligand analogue T4-FITC for binding sites on a population of enzyme-labelled antibodies specific for T4. A simultaneous incubation protocol is used and magnetisable particles carrying antibody specific for FITC are used for separation of bound and free conjugate.

Preparation of starting materials
(i) Assay buffer
75 mM Barbitone containing 0.02% (w/v) bovine serum albumin, pH 8.6.

(ii) Labelled antibodies to thyroxine
Monoclonal antibodies to thyroxine were obtained from mouse ascites fluid by a process based on that originally reported by Milstein and Kohler in Nature *256* 495—497 (1975).

Monoclonal antibodies were conjugated to β-galactosidase using a heterobifunctional reagent by methods known to those versed in the art (see, for example, Ishikawa et al, in J. Immunoassay *4* 209—327 (1983)). The conjugates were purified by HPLC using TSK 4000 sw columns and diluted in the assay buffer.

(iii) FITC-T4
This material was prepared and purified according to the method of Smith in FEBS Letters *77* 25 (1977) and diluted in assay buffer to 0.2 µg/ml.

(iv) Solid phase reagent
This material comprised anti-FITC polyclonal antibody covalently linked to magnetisable cellulose particles.

Anti-FITC was a conventional polyclonal antiserum obtained by immunising sheep with FITC conjugated to keyhole limpet haemocyanin. The magnetisable cellulose particles were a composite of cellulose containing approximately 50% black ferric(ous) oxide ($Fe_3O_4$), with mean particle diameter of 3 micron (see Forrest and Rattle, "Magnetic Particle Radio Immunoassay", in "Immunoassays for Clinical Chemistry", p. 147—162, Ed. Hunter and Corrie, Churchill, Livingstone, Edinburgh (1982)). Anti-FITC antiserum was covalently coupled to the magnetisable cellulose following cyanogen bromide activation of the cellulose, according to the procedure of Axen et al., Nature *214*, 1302—1304 (1967). The antiserum was coupled at a ratio of 2 ml antiserum to 1 gram of magnetisable solid phase.

Anti-FITC magnetisable solid phase was diluted to a concentration of 6—8 mg/ml in sodium phosphate buffer 0.05M, pH 7.4 containing 0.25% (w/v) bovine serum albumin, 0.25% (v/v) Triton X-100, 0.8% (w/v) hydroxypropyl methyl cellulose and 0.1% (w/v) sodium azide.

(v) Substrate
0.127% (w/v) p-nitrophenyl-β-D-galactopyranoside in 20 mM tris/HCl pH 7.4 containing 150 mM sodium chloride and 1 mM magnesium chloride.

(vi) Stop solution
200 mM tris.

Determination of thyroxine

Standards were prepared by adding known concentrations of thyroxine to charcoal-stripped normal human serum.

Duplicate samples were run. To 200 µl of standard were added 200 µl of assay buffer containing 0.2% (w/v) 8-anilino-1-naphthalenesulphonic acid (ANS), 50 µl of T4-FITC solution and 50 µl of enzyme-antibody conjugate solution.

After incubation of the mixture for 1 hour at ambient temperature, 200 µl of the solid-phase suspension were added. After mixing, the mixture was incubated for a further 15 minutes. The solid-phase particles were then separated magnetically, the liquid removed by decantation and the particles washed by addition of 500 µl of buffer. Following this, the particles were again separated magnetically and the wash liquid removed by decantation.

The enzyme activity associated with the resultant pellet was detected by contacting the pellet with 100 µl of substrate and incubating for 30 minutes at 37°C. The reaction with the substrate was terminated by the addition of 1 ml of stop solution. After sedimenting the particles magnetically, the absorbance of the supernatant at 404 nm was measured spectrophotometrically.

Below are the data obtained from assays performed using standards containing different concentrations of thyroxine. The data are means of duplicates; the non-specific binding has been subtracted.

| Concentration of thyroxine (ng/ml) | Absorbance (OD404) |
|---|---|
| 0 | 1.037 |
| 5 | 0.713 |
| 50 | 0.198 |
| 500 | 0.025 |

Example 2
Enzyme immunoassay of oestradiol (E2)

In this assay, $E_2$ in the sample competes with the link ligand analogue $E_2$-BSA-FITC for binding sites on a population of enzyme-labelled antibodies specific for $E_2$. A simultaneous incubation protocol is used and magnetisable particles carrying antibody specific for FITC are used for separation of bound and free conjugate.

Preparation of starting materials
(i) Assay buffer

The assay buffer was 100 mM tris-HCl pH 8.0 containing 0.5% (w/v) BSA, 0.2% (v/v) sheep serum, 1 mM $MgCl_2$, 0.1 mM $ZnCl_2$, 100 mM NaCl and 0.2 (w/v) $NaN_3$.

(ii) Labelled antibodies to oestradiol

Monoclonal antibodies to $E_2$ were obtained from mouse ascites fluid by a process based on that originally reported by Milstein and Kohler in Nature *256* 495—497 (1975).

Monoclonal antibodies were conjugated to alkaline phosphatase using a heterobifunctional reagent by methods known to those versed in the art (see, for example, Ishikawa et al., in J. Immunoassay *4* 209—327 (1983)). The conjugates were purifed by HPLC using TSK 3000sw columns and diluted in assay buffer.

(iii) $E_2$-BSA-FITC

Oestradiol 17 B-hemisuccinate: BSA-FITC conjugate was obtained from Sigma. The material contains 4—9 moles of steroid per mole of protein and 2—5 moles of FITC per mole of steroid-BSA conjugate. This material was diluted in assay buffer to 50 ng/ml.

(iv) Solid phase reagent
As in Example 1 (iv).

(v) Substrate

This was 15 mM p-nitrophenyl phosphate in 1M diethanolamine buffer pH 8.6 containing 0.9% (w/v) sodium chloride and 1 mM magnesium chloride.

(vi) Stop solution

This was a solution containing 200 mM EDTA tetrasodium salt, 200 mM sodium carbonate, 20 mM trisodium phosphate and 100 mM sodium hydroxide.

Determination of oestradiol

Standards were prepared by adding known concentrations of oestradiol 17B to 50 mM phosphate buffer pH 7.4 containing 0.9% (w/v) sodium chloride.

Duplicate samples were run. To 100 µl of standard, 50 µl of enzyme-antibody solution was added.

After incubation of the mixture for 30 minutes at 37°C, 200 µl of the solid phase suspension was added. After mixing, the mixture was incubated for 5 minutes at 37°C. The solid-phase particles were then separated magnetically, the liquid removed by decantation and the particles washed by addition of 100 µl of buffer. The particles were again separated magnetically and the wash liquid removed by decantation. The wash process was repeated twice.

The enzyme activity associated with the resultant pellet was detected by contacting the pellet with 300 µl of substrate and incubating for 10 minutes at 37°C. The reaction with the substrate was terminated by the addition of 1 ml of stop solution. After sedimenting the particles magnetically, the absorbance of the supernatant at 404 nm was measured spectrophotometrically.

Below are the data obtained from assays performed using standards containing different concentrations of oestradiol. The data are means of duplicates; the non-specific binding has been subtracted.

| Concentration of oestradiol (pg/ml) | Absorbance (OD404) |
| --- | --- |
| 0 | 0.761 |
| 5 | 0.711 |
| 10 | 0.710 |
| 25 | 0.663 |
| 50 | 0.660 |
| 100 | 0.480 |
| 250 | 0.395 |
| 500 | 0.197 |
| 1000 | 0.102 |
| 2000 | 0.045 |

## Claims

1. A method of assay of a ligand in a sample, which comprises incubating, simultaneously or in sequence,

(a) the sample (containing the analyte ligand),

(b) a specific binding partner to the ligand, the said specific binding partner being directly labelled and not being part of a complex with a labelled specific binding partner therefor and

(c) a ligand analogue as herein defined, conjugated with a reagent X (the said reagent not being present as a free reagent in the mixture);

separating, after a suitable incubation period, the portion containing component (c) from the mixture by means of a solid phase carrying a binder partner specific for reagent X; and determining the extent of complexing between components (b) and (c) by measurement of the label.

2. A method as claimed in claim 1 wherein the analyte ligand is a hapten.

3. A method as claimed in claim 2 wherein the analyte ligand is a non-peptide hormone or metabolite thereof.

4. A method as claimed in any one of claims 1 to 3 wherein component (b) is a directly-labelled antibody.

5. A method as claimed in claim 4 wherein component (b) is a directly-labelled monoclonal antibody.

6. A method as claimed in any one of claims 1 to 3 wherein component (b) is a directly-labelled monoclonal or polyclonal antibody fragment devoid of the Fc portion.

7. A method as claimed in any one of claims 1 to 6 wherein reagent X is fluorescein isothiocyanate, rhodamine isothiocyanate, 2,4-dinitrofluorobenzene, phenyl isothiocyanate or dansyl chloride.

8. A method as claimed in any one of claims 1 to 7 wherein reagent X and the ligand analogue in component (c) are separated by a spacer.

9. A method as claimed in claim 8 wherein the spacer is selected from proteins, peptides, oligosaccharides, polysaccharides and synthetic polymers.

10. A method as claimed in any one of claims 1 to 9 wherein the solid phase comprises magnetisable particles.

11. A kit of reagents for carrying out a method of assay as claimed in claim 1 which comprises components (b) and (c) and a solid phase carrying a binding partner specific for reagent X.

## Patentansprüche

1. Verfahren zur Bestimmung eines Liganden in einer Probe, dadurch gekennzeichnet, daß gleichzeitig oder aufeinanderfolgend inkubiert werden

(a) die Probe (enthaltend den Analyt-Liganden),

(b) ein spezifischer Bindungspartner für den Liganden, wobei dieser spezifische Bindungspartner direkt markiert ist und nicht Teil eines Komplexes mit einem markierten, spezifischen Bindungspartner dafür ist, und

(c) ein Ligandenanaloges, wie hier definiert, das konjugiert ist mit einem Reagens X (wobei dieses Reagents nicht als freies Reagens in dem Gemisch vorhanden ist);

Abtrennen des die Komponente (c) enthaltenden Teils nach einer geeigneten Inkubationszeit aus dem Gemisch mittels einer festen Phase, die einen Bindungspartner, spezifisch für das Reagens X, trägt;

und Bestimmung des Ausmaßes der Komplexierung zwischen den Komponenten (b) und (c) durch Messung des Labels (Markierung).

2. Verfahren gemäß Anspruch 1, dadurch gekennzeichnet, daß der Analyt-Ligand ein Hapten ist.

3. Verfahren gemäß Anspruch 2, dadurch gekennzeichnet, daß der Analyt-Ligand ein Nicht-Peptid-Hormon oder ein Metabolit davon ist.

4. Verfahren gemäß einem der Ansprüche 1 bis 3, dadurch gekennzeichnet, daß die Komponente (b) ein direkt markierter Antikörper ist.

5. Verfahren gemäß Anspruch 4, dadurch gekennzeichnet, daß die Komponente (b) ein direkt markierter, monoklonaler Antikörper ist.

6. Verfahren gemäß einem der Ansprüche 1 bis 3, dadurch gekennzeichnet, daß die Komponente (b) ein direkt markiertes, monoklonales oder polyklonales Antikörper-Fragment ohne den Fc-Teil ist.

7. Verfahren gemäß einem der Ansprüche 1 bis 6, dadurch gekennzeichnet, daß das Reagens X Fluoresceinisothiocyanat, Rhodamin-isothiocyanat, 2,4-Dinitrofluorbenzol, Phenylisothiocyanat oder Dansylchlorid ist.

8. Verfahren gemäß einem der Ansprüche 1 bis 7, dadurch gekennzeichnet, daß das Reagens X und das Ligandenanaloge in Komponente (c) durch einen Spacer getrennt sind.

9. Verfahren gemäß Anspruch 8, dadurch gekennzeichnet, daß das Spacer ausgewählt ist aus Proteinen, Peptiden, Oligosacchariden, Polysacchariden und synthetischen Polymeren.

10. Verfahren gemäß einem der Ansprüche 1 bis 9, dadurch gekennzeichnet, daß die feste Phase magnetisierbare Teilchen umfaßt.

11. Reagens-Kit zur Durchführung einer Bestimmungsmethode gemäß Anspruch 1, umfassend die Komponenten (b) und (c) und eine feste Phase, die einen Bindungspartner, spezifisch für das Reagens X, trägt.

**Revendications**

1. Procédé de dosage d'un ligand dans un échantillon, qui comprend l'incubation, simultanément ou en succession,

(a) de l'échantillon (contenant le ligand à analyse),

(b) d'un partenaire de fixation spécifique pour le ligand, ce partenaire de fixation spécifique étant marqué directement et ne faisant pas partie d'un complexe avec un partenaire de fixation spécifique marqué pour celui-ci, et

(c) d'un analogue de ligand tel que défini ici, conjugué à un réactant X (ce réactant n'étant pas présent à l'état de réactant libre dans le mélange);

la séparation, après une durée d'incubation appropriée, de la fraction contenant le composant (c) hors du mélange à l'aide d'une phase solide portant un partenaire de fixation spécifique pour le réactant X, et

la détermination du degré de complexation entre les composants (b) et (c) par mesure du marqueur.

2. Procédé suivant la revendication 1, dans lequel le ligand à analyser est un haptène.

3. Procédé suivant la revendication 2, dans lequel le ligand à analyser est une hormone non peptidique ou un métabolite de celle-ci.

4. Procédé suivant l'une quelconque des revendications 1 à 3, dans lequel le composant (b) est un anticorps directement marqué.

5. Procédé suivant la revendication 4, dans lequel le composant (b) est un anticorps monoclonal directement marqué.

6. Procédé suivant l'une quelconque des revendications 1 à 3, dans lequel le composant (b) est un fragment d'anticorps monoclonal ou polyclonal directement marqué exempt de partie Fc.

7. Procédé suivant l'une quelconque des revendications 1 à 6, dans lequel le réactant X est l'isothiocyanate de fluorescéine, l'isothiocyanate de rhodamine, le 2,4-dinitrofluorobenzène, l'isothiocyanate de phényle ou le chlorure de dansyle.

8. Procédé suivant l'une quelconque des revendications 1 à 7, dans lequel le réactant X et l'analogue de ligand dans le composant (c) sont séparés par un écarteur.

9. Procédé suivant la revendication 8, dans lequel l'écarteur est choisi parmi les protéines, les peptides, les oligosaccharides, les polysaccharides et les polymères synthétiques.

10. Procédé suivant l'une quelconque des revendications 1 à 9, dans lequel la phase solide comprend des particules magnétisables.

11. Trousses de réactants pour l'exécution d'un procédé de dosage suivant la revendication 1, qui comprend les composants (b) et (c) et une phase solide portant un partenaire de fixation spécifique pour le réactant X.